# EUROPÄISCHE PATENTANMELDUNG

(11) **EP 2 361 603 A2**
(43) Veröffentlichungstag der Anmeldung: **31.08.2011**
(21) Anmeldenummer: 10187416.2
(22) Anmeldetag: 13.10.2010
(51) Int. Cl.: A61K 8/19, A61K 8/44, A61Q 11/00

(54) **Desensibilisierende Mund- und Zahnpflege- und -Reinigungsmittel mit Ethyllaroylarginat**

(30) Priorität: 22.02.2010 DE 102010002194
(71) Anmelder: Henkel AG & Co. KGaA, 40589 Düsseldorf (DE)
(72) Erfinder: Adomat, Christel, 40591, Düsseldorf (DE); Barth, Adolf Peter, 42781, Haan (DE); Leinen, Hans-Theo, 40699, Erkrath (DE)

(57) **Zusammenfassung**

Mund- und Zahnpflege- und -reinigungsmittel mit besonders hoher desensibilisierender, antibakterieller und plaquereduzierender Wirkung enthalten bezogen auf ihr Gewicht in einem oral akzeptablen Träger 0,01 bis 2,5 Gew.-% Ethyllauroylarginat und 0,1 bis 10,0 Gew.-% mindestens eines Kalium- oder Strontiumsalzes.

## Beschreibung

Die Erfindung betrifft Mund- und Zahnpflegemittel mit einer Wirkstoffkombination zur Verringerung der Sensibilität der Zähne gegen mechanische und thermische Reize, die gleichzeitig die Zahnoberflächen gründlich reinigt und Mundgeruch verringern hilft.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern. Es hat nicht an Versuchen gefehlt, Mundgeruch zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung.

Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können.

Es besteht daher nach wie vor das Bedürfnis, Halitosis zu bekämpfen und Wirkstoffe oder Wirkstoffkombinationen dafür bereitzustellen, die frei von den genannten Nachteilen sind.

Manche Menschen leiden unter einer extremen Empfindlichkeit der Zähne, insbesondere im Bereich der Zahnhälse und verspüren ein unangenehmes Gefühl bis hin zum Schmerz, wenn die Zähne z.B. beim Genuß gekühlter Speisen oder Getränke oder bei der Berührung mit der Zahnbürste einem thermischen oder mechanischen Reiz ausgesetzt werden. Es ist bekannt, daß wasserlösliche Salze des Kaliums und Strontiums diese Unempfindlichkeit in gewissem Umfang verringern können. So wird z.B. in DE 24 19 384 ein Zusatz von Kaliumnitrat, in US 3,122,483 der Zusatz von Strontiumchlorid zu Zahnpasten empfohlen.

Um die Einsatzmenge herkömmlicher antibakterieller Mittel zu verringern, werden oftmals synergistische Kombinationen mehrerer antibakterieller Mittel eingesetzt. So schlägt die WO 03/043 593 A1 vor, herkömmliche antibakterielle Substanzen wie Triclosan, Phenoxyethanol oder Hexetidin mit Ethyllauroylarginat zu kombinieren, um die antibakterielle Wirkung zu verstärken. Der Einsatz von Ethyllauroylarginat in Zahnpasten oder als Agens gegen Plaque in Bonbons oder Kaugummis ist beispielsweise aus den Offenlegungsschriften WO 03/013 453 A1**,** WO 03/013 454 A1 und US-A 2004/258629 bekannt.

Die Verwendung von Ethyllaurylarginat zum Schutz vor Erosion an Zahnoberflächen wird in der WO 2009/101115 A1 beschrieben.

Auch wenn durch den verringerten Einsatz von Triclosan die geschmacklichen Probleme sowie Risiken der Verfärbung von Zahnoberflächen minimiert werden, besteht nach wie vor das Bedürfnis, Wirkstoffe oder Wirkstoffkombinationen bereitzustellen, die sowohl frei von den genannten Nachteilen sind als auch einen Zusatznutzen beinhalten.

Die Aufgabe der vorliegenden Erfindung bestand darin, Zubereitungen zur Mund- und Zahnpflege und -reinigung bereitzustellen, die die Zähne desensibilisieren und zudem Mundgeruch wirksam bekämpfen und gegen Gingivitis und Parodontitis wirksam sind.

Es wurde nun aber gefunden, daß ein Gehalt an Ethyllauroylarginat die desensibilisierende Wirkung von Kaliumsalzen auf überempfindliche Zähne noch erheblich steigert. Insbesondere wurde ein rascheres Eintreten der Wirkung beobachtet. Eine solche, im Zusammenwirken mit anderen desensibilisierend wirkenden Stoffen potenzierende Wirkung des Ethyllauroylarginats war bisher unbekannt.

Gegenstand der Erfindung sind daher Mund- und Zahnpflege- und -reinigungsmittel, enthaltend - bezogen auf sein Gewicht -
a) 0,01 bis 2,5 Gew.-% Ethyllauroylarginat
b) 0,1 bis 10,0 Gew.-% mindestens eines Kalium- oder Strontiumsalzes.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Eine besonders bevorzugte Ausführungsform der vorliegenden Erfindung sind Mundspüllösungen und Mundwasser, die zum Ausspülen der Mundhöhle verwendet werden.

Die erfindungsgemäßen Mittel enthalten als ersten Inhaltsstoff 0,01 bis 2,5 Gew.-% Ethyllauroylarginat (ELA bzw. LAE) 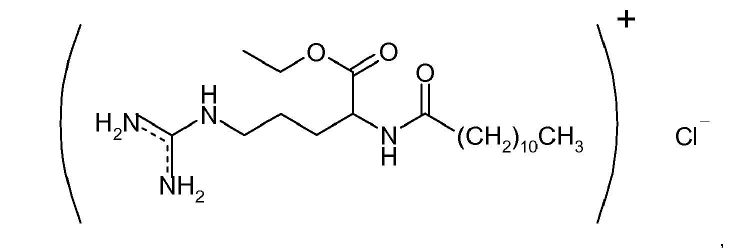 das auch als Ethyl-N^{alpha}-dodecanoyl-L-Arginat Hydrochlorid bezeichnet wird. LAE ist kommerziell erhältlich und gilt als unbedenklich, auch für den Einsatz in Nahrungsmitteln.

Erfindungsgemäß bevorzugte Mund- und Zahnpflege- und -reinigungsmittel enthalten - bezogen auf ihr Gewicht - - 0,05 - 2,25 Gew.-%, vorzugsweise 0,075 - 2 Gew.-%, besonders bevorzugt 0,1 - 1,75 Gew.-%, außerordentlich bevorzugt 0,125 - 1,5 Gew.-% und insbesondere 0,15 bis 0,8 Gew.-% Ethyllauroylarginat.

Als weiteren zwingenden Inhaltsstoff enthalten die erfindungsgemäßen Mund- und Zahnpflege-und -reinigungsmittel 0,1 bis 10,0 Gew.-% mindestens eines Kaliumsalzes.

Der Einsatz der Kaliumsalze innerhalb engerer Mengenbereiche ist bevorzugt. Bevorzugte erfindungsgemäße Mund und Zahnpflege- und -reinigungsmittel sind dadurch gekennzeichnet, daß sie 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e) oder Strontiumsalz(e) enthalten.

Obwohl hinsichtlich der Auswahl der Kaliumsalze keine Einschränkungen bestehen, sind bevorzugte Kaliumsalze gut wasserlöslich. Besonders bevorzugte erfindungsgemäße Mund und Zahnpflege- und -reinigungsmittel sind dabei dadurch gekennzeichnet, daß sie ausschließlich Kaliumsalz(e) enthalten, welche(s) bei 20°C eine Löslichkeit in destilliertem Wasser oberhalb von 200 g/l, vorzugsweise oberhalb von 250 g/l und insbesondere oberhalb von 300 g/l aufweisen. Geeignete wasserlösliche Salze des Kaliums sind z.B. Kaliumchlorid, Kaliumsulfat, Kaliumbicarbonat, Kaliumcitrat, Kaliumacetat, Kaliumlactat und Kaliumnitrat. Bevorzugt eignet sich Kaliumnitrat.

Es hat sich gezeigt, daß die desensibilisierende Wirkung von Kaliumsalzen auf überempfindliche Zähne durch den Einsatz von Ethyllauroylarginat gesteigert wird. Die Steigerung ist besonders innerhalb bestimmte Gewichtsverhältnisse der Komponenten zueinander zu beobachten. Bei bevorzugten erfindungsgemäßen Mund und Zahnpflege- und -reinigungsmitteln beträgt das Gewichtsverhältnis zwischen Ethyllauroylarginat und der Gesamtmenge an Kaliumsalz(en) 1 : 1000 bis 1 : 1, vorzugsweise 0,5 : 100 bis 75 : 100, weiter bevorzugt 1 : 100 bis 50 : 100,noch weiter bevorzugt 1,5 : 100 bis 10 : 100 und insbesondere 2 : 100 bis 3 : 100.

Besonders bevorzugte erfindungsgemäße Mittel enthalten 0,15 bis 0,8 Gew.-% Ethyllauroylarginat und 4 bis 6 Gew.-% Kaliumnitrat, wobei Mittel, die folgende Kombinationen enthalten, besonders bevorzugt sind:

| **Linolsäure (< 1% sonstige Ölsäuren).** **[Gew.-%, bezogen auf gesamtes Mittel]** | **Kaliumnitrat** **[Gew.-%, bezogen auf gesamtes Mittel]** |
|---|---|
| 0,15 | 4,0 |
| 0,15 | 4,1 |
| 0,15 | 4,2 |
| 0,15 | 4,3 |
| 0,15 | 4,4 |
| 0,15 | 4,5 |
| 0,15 | 4,6 |
| 0,15 | 4,7 |
| 0,15 | 4,8 |
| 0,15 | 5,0 |
| 0,15 | 5,1 |
| 0,15 | 5,2 |
| 0,15 | 5,3 |
| 0,15 | 5,4 |
| 0,15 | 5,5 |
| 0,15 | 5,6 |
| 0,15 | 5,7 |
| 0,15 | 5,8 |
| 0,15 | 5,9 |
| 0,15 | 6,0 |
| 0,20 | 4,0 |
| 0,20 | 4,1 |
| 0,20 | 4,2 |
| 0,20 | 4,3 |
| 0,20 | 4,4 |
| 0,20 | 4,5 |
| 0,20 | 4,6 |
| 0,20 | 4,7 |
| 0,20 | 4,8 |
| 0,20 | 5,0 |
| 0,20 | 5,1 |
| 0,20 | 5,2 |
| 0,20 | 5,3 |
| 0,20 | 5,4 |
| 0,20 | 5,5 |
| 0,20 | 5,6 |
| 0,20 | 5,7 |
| 0,20 | 5,8 |
| 0,20 | 5,9 |
| 0,20 | 6,0 |
| 0,25 | 4,0 |
| 0,25 | 4,1 |
| 0,25 | 4,2 |
| 0,25 | 4,3 |
| 0,25 | 4,4 |
| 0,25 | 4,5 |
| 0,25 | 4,6 |
| 0,25 | 4,7 |
| 0,25 | 4,8 |
| 0,25 | 5,0 |
| 0,25 | 5,1 |
| 0,25 | 5,2 |
| 0,25 | 5,3 |
| 0,25 | 5,4 |
| 0,25 | 5,5 |
| 0,25 | 5,6 |
| 0,25 | 5,7 |
| 0,25 | 5,8 |
| 0,25 | 5,9 |
| 0,25 | 6,0 |
| 0,30 | 4,0 |
| 0,30 | 4,1 |
| 0,30 | 4,2 |
| 0,30 | 4,3 |
| 0,30 | 4,4 |
| 0,30 | 4,5 |
| 0,30 | 4,6 |
| 0,30 | 4,7 |
| 0,30 | 4,8 |
| 0,30 | 5,0 |
| 0,30 | 5,1 |
| 0,30 | 5,2 |
| 0,30 | 5,3 |
| 0,30 | 5,4 |
| 0,30 | 5,5 |
| 0,30 | 5,6 |
| 0,30 | 5,7 |
| 0,30 | 5,8 |
| 0,30 | 5,9 |
| 0,30 | 6,0 |
| 0,35 | 4,0 |
| 0,35 | 4,1 |
| 0,35 | 4,2 |
| 0,35 | 4,3 |
| 0,35 | 4,4 |
| 0,35 | 4,5 |
| 0,35 | 4,6 |
| 0,35 | 4,7 |
| 0,35 | 4,8 |
| 0,35 | 5,0 |
| 0,35 | 5,1 |
| 0,35 | 5,2 |
| 0,35 | 5,3 |
| 0,35 | 5,4 |
| 0,35 | 5,5 |
| 0,35 | 5,6 |
| 0,35 | 5,7 |
| 0,35 | 5,8 |
| 0,35 | 5,9 |
| 0,35 | 6,0 |
| 0,40 | 4,0 |
| 0,40 | 4,1 |
| 0,40 | 4,2 |
| 0,40 | 4,3 |
| 0,40 | 4,4 |
| 0,40 | 4,5 |
| 0,40 | 4,6 |
| 0,40 | 4,7 |
| 0,40 | 4,8 |
| 0,40 | 5,0 |
| 0,40 | 5,1 |
| 0,40 | 5,2 |
| 0,40 | 5,3 |
| 0,40 | 5,4 |
| 0,40 | 5,5 |
| 0,40 | 5,6 |
| 0,40 | 5,7 |
| 0,40 | 5,8 |
| 0,40 | 5,9 |
| 0,40 | 6,0 |
| 0,45 | 4,0 |
| 0,45 | 4,1 |
| 0,45 | 4,2 |
| 0,45 | 4,3 |
| 0,45 | 4,4 |
| 0,45 | 4,5 |
| 0,45 | 4,6 |
| 0,45 | 4,7 |
| 0,45 | 4,8 |
| 0,45 | 5,0 |
| 0,45 | 5,1 |
| 0,45 | 5,2 |
| 0,45 | 5,3 |
| 0,45 | 5,4 |
| 0,45 | 5,5 |
| 0,45 | 5,6 |
| 0,45 | 5,7 |
| 0,45 | 5,8 |
| 0,45 | 5,9 |
| 0,45 | 6,0 |
| 0,50 | 4,0 |
| 0,50 | 4,1 |
| 0,50 | 4,2 |
| 0,50 | 4,3 |
| 0,50 | 4,4 |
| 0,50 | 4,5 |
| 0,50 | 4,6 |
| 0,50 | 4,7 |
| 0,50 | 4,8 |
| 0,50 | 5,0 |
| 0,50 | 5,1 |
| 0,50 | 5,2 |
| 0,50 | 5,3 |
| 0,50 | 5,4 |
| 0,50 | 5,5 |
| 0,50 | 5,6 |
| 0,50 | 5,7 |
| 0,50 | 5,8 |
| 0,50 | 5,9 |
| 0,50 | 6,0 |
| 0,55 | 4,0 |
| 0,55 | 4,1 |
| 0,55 | 4,2 |
| 0,55 | 4,3 |
| 0,55 | 4,4 |
| 0,55 | 4,5 |
| 0,55 | 4,6 |
| 0,55 | 4,7 |
| 0,55 | 4,8 |
| 0,55 | 5,0 |
| 0,55 | 5,1 |
| 0,55 | 5,2 |
| 0,55 | 5,3 |
| 0,55 | 5,4 |
| 0,55 | 5,5 |
| 0,55 | 5,6 |
| 0,55 | 5,7 |
| 0,55 | 5,8 |
| 0,55 | 5,9 |
| 0,55 | 6,0 |
| 0,60 | 4,0 |
| 0,60 | 4,1 |
| 0,60 | 4,2 |
| 0,60 | 4,3 |
| 0,60 | 4,4 |
| 0,60 | 4,5 |
| 0,60 | 4,6 |
| 0,60 | 4,7 |
| 0,60 | 4,8 |
| 0,60 | 5,0 |
| 0,60 | 5,1 |
| 0,60 | 5,2 |
| 0,60 | 5,3 |
| 0,60 | 5,4 |
| 0,60 | 5,5 |
| 0,60 | 5,6 |
| 0,60 | 5,7 |
| 0,60 | 5,8 |
| 0,60 | 5,9 |
| 0,60 | 6,0 |
| 0,65 | 4,0 |
| 0,65 | 4,1 |
| 0,65 | 4,2 |
| 0,65 | 4,3 |
| 0,65 | 4,4 |
| 0,65 | 4,5 |
| 0,65 | 4,6 |
| 0,65 | 4,7 |
| 0,65 | 4,8 |
| 0,65 | 5,0 |
| 0,65 | 5,1 |
| 0,65 | 5,2 |
| 0,65 | 5,3 |
| 0,65 | 5,4 |
| 0,65 | 5,5 |
| 0,65 | 5,6 |
| 0,65 | 5,7 |
| 0,65 | 5,8 |
| 0,65 | 5,9 |
| 0,65 | 6,0 |
| 0,70 | 4,0 |
| 0,70 | 4,1 |
| 0,70 | 4,2 |
| 0,70 | 4,3 |
| 0,70 | 4,4 |
| 0,70 | 4,5 |
| 0,70 | 4,6 |
| 0,70 | 4,7 |
| 0,70 | 4,8 |
| 0,70 | 5,0 |
| 0,70 | 5,1 |
| 0,70 | 5,2 |
| 0,70 | 5,3 |
| 0,70 | 5,4 |
| 0,70 | 5,5 |
| 0,70 | 5,6 |
| 0,70 | 5,7 |
| 0,70 | 5,8 |
| 0,70 | 5,9 |
| 0,70 | 6,0 |
| 0,75 | 4,0 |
| 0,75 | 4,1 |
| 0,75 | 4,2 |
| 0,75 | 4,3 |
| 0,75 | 4,4 |
| 0,75 | 4,5 |
| 0,75 | 4,6 |
| 0,75 | 4,7 |
| 0,75 | 4,8 |
| 0,75 | 5,0 |
| 0,75 | 5,1 |
| 0,75 | 5,2 |
| 0,75 | 5,3 |
| 0,75 | 5,4 |
| 0,75 | 5,5 |
| 0,75 | 5,6 |
| 0,75 | 5,7 |
| 0,75 | 5,8 |
| 0,75 | 5,9 |
| 0,75 | 6,0 |
| 0,80 | 4,0 |
| 0,80 | 4,1 |
| 0,80 | 4,2 |
| 0,80 | 4,3 |
| 0,80 | 4,4 |
| 0,80 | 4,5 |
| 0,80 | 4,6 |
| 0,80 | 4,7 |
| 0,80 | 4,8 |
| 0,80 | 5,0 |
| 0,80 | 5,1 |
| 0,80 | 5,2 |
| 0,80 | 5,3 |
| 0,80 | 5,4 |
| 0,80 | 5,5 |
| 0,80 | 5,6 |
| 0,80 | 5,7 |
| 0,80 | 5,8 |
| 0,80 | 5,9 |
| 0,80 | 6,0 |

Alternativ oder zusätzlich zu den Kaliumsalzen können die erfindungsgemäßen Mittel auch Strontiumsalze enthalten. Geeignete wasserlösliche Salze des Strontiums sind Strontiumchlorid, Strontiumnitrat, Strontiumcitrat, Strontiumacetat und Strontiumlactat.

Die erfindungsgemäße Kombination gegen empfindliche Zähne ist dann besonders effektiv, wenn auch der Wassergehalt der erfindungsgemäßen Mittel speziell auf sie abgestimmt wird. Insbesondere in wasserärmeren Formulierungen ist der Effekt der Desensibilisierung besonders ausgeprägt. Hier sind bevorzugte erfindungsgemäße Mund und Zahnpflege- und -reinigungsmittel dadurch gekennzeichnet, daß sie weniger als 60 Gew.-%, vorzugsweise weniger als 55 Gew.-%, besonders bevorzugt weniger als 50 Gew.-% und insbesondere weniger als 45 Gew.-% Wasser enthalten.

Die erfindungsgemäßen Mittel sind an sich bereits wirksam gegen Mundgeruch (Halitosis) sowie Parodontitis und/oder Gingivitis. Zur Steigerung dieser Effekte können sie weitere entzündungshemmende Substanzen enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2`-hydroxydiphenylether, 4,4`-Dichlor-2`-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

Bevorzugte erfindungsgemäße Mund und Zahnpflege- und -reinigungsmittel enthalten zur Verbesserung der entzündungshemmenden Eigenschaften eine Kombination von
- Panthenol oder einem Salz der Pantothensäure und
- Retinol oder einem Derivat davon.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-ß-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ 1.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ 1.E. (Internationale Einheiten) pro 100 g entspricht.

Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mund- und Zahnpflege- und -reinigungsmittel enthalten weitere Inhaltsstoffe. Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel. Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(11)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Der Einsatz von Putzkörpern (Abrasiva) ist in den Zahnpasten unter den erfindungsgemäßen Mund- und Zahnpflege- und -reinigungsmittel ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 ― 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 — 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 ― 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.- % enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind solche Zahnpasten unter den erfindungsgemäßen Mund- und Zahnpflege-und -reinigungsmitteln bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ -2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O7_{,} Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.

Geeignete Tenside sind z. B. lineare Natriumalkylsulfate mit 12―18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12―16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8―18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside sowie Fettsäureamidoalkylbetaine.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Ein weiterer Gegenstand der vorliegenden Erfindung ist die Verwendung eines erfindungsgemäßen Mund und Zahnpflege- und -reinigungsmittels zur
- Senkung der Zahnempfindlichkeit und/oder
- Senkung der Zahnempfindlichkeit gegen süße Speisen und Getränke und/oder
- Senkung der Zahnempfindlichkeit gegen saure Speisen und Getränke und/oder
- Senkung der Zahnempfindlichkeit gegen heiße Speisen und Getränke und/oder
- Senkung der Zahnempfindlichkeit gegen kalte Speisen und Getränke und/oder
- Senkung der Zahnempfindlichkeit gegen taktile Reize.

Bezüglich bevorzugter Ausführungsformen der erfindungsgemäßen Verwendung gilt mutatis mutandis das zu den erfindungsgemäßen Mitteln Gesagte.

Ein weiterer Gegenstand der vorliegenden Erfindung ist ein Verfahren zur Desensibilisierung empfindlicher Zähne, dadurch gekennzeichnet, daß ein erfindungsgemäßes Mund und Zahnpflege-und -reinigungsmittel in Form einer Zahnpasta konfektioniert und zum Putzen der Zähne mittels einer Zahnbürste eingesetzt wird oder in Form eines Mundwassers konfektioniert ist und zum Spülen der Mundhöhle verwendet wird..

Auch bezüglich bevorzugter Ausführungsformen der erfindungsgemäßen Verfahren gilt mutatis mutandis das zu den erfindungsgemäßen Mitteln Gesagte.

### Beispiele:

### Mundspüllösungen (alle Angaben in Gew.-%):

| | **M1** | **M2** | **M3** | **M4** | **M5** | **M6** | **M7** | **M8** |
|---|---|---|---|---|---|---|---|---|
| Sorbitol 70% | 3,0 | 3 | 0 | 3 | 0 | 3 | 0 | 3 |
| Ethyllauroylarginat | 0,1 | 0,2 | 0,3 | 0,4 | 0,5 | 0,6 | 0,7 | 0,8 |
| Kaliumnitrat | 5,0 | 5,0 | 5,0 | 5,0 | 5,0 | 5,0 | 5,0 | 5,0 |
| Natriumfluorid | 0,05 | 0,05 | 0,05 | 0,05 | 0,05 | 0,045 | 0,045 | 0,045 |
| Saccharin Natrium | 0,03 | 0,03 | 0,03 | 0,03 | 0,03 | 0,03 | 0,03 | 0,03 |
| Capryl/Caprylyl Glucosid (70% Aktivsubstanz) | 0,86 | 0,86 | 0,86 | 0,86 | 0,86 | 0,86 | 0,86 | 0,86 |
| Aroma | 0,1 | 0,1 | 0,1 | 0,1 | 0,1 | 0,1 | 0,1 | 0,1 |
| Wasser, vollentsalzt | ad 100 | ad 100 | ad 100 | ad 100 | ad 100 | ad 100 | ad 100 | ad 100 |

Es wurden Zahnpasten folgender Zusammensetzung (alle Angaben in Gew.-%)hergestellt:

| | **E1** | **E2** | **E3** | **E4** | **E5** |
|---|---|---|---|---|---|
| Sorbit (70 % DAB) | 60 | 62,5 | 65 | 67,5 | 70,0 |
| Ethanol (96%) | - | - | - | - | 2,0 |
| Fällungskieselsäure : Sident 8 | 10,0 | 10,0 | 10,0 | 10,0 | 12,0 |
| Fällungskieselsäure : Sident 22S | 8,5 | 8,5 | 8,5 | 8,5 | - |
| Poliertonerde P10 feinst | - | 1,0 | - | - | - |
| Dinatriumphosphat, wasserfrei | 0,1 | 0,1 | 0,1 | 0,1 | 0,1 |
| Trinatriumphosphat, wasserfrei | 0,2 | 0,2 | 0,2 | 0,2 | - |
| Na-Monofluorophosphat Na2P03F | - | - | 1,0 | 1,0 | 1,0 |
| Natriumfluorid | 0,32 | 0,32 | - | - | - |
| Polyethylenglycol (MG : 1500) | 1,5 | 1,5 | 1,5 | 1,5 | 1,5 |
| Titandioxid | 0,5 | 0,5 | 0,5 | 0,5 | - |
| Na-Laurylsulfat | 1,5 | 1,5 | 1,5 | 1,5 | 1,5 |
| Saccharin-Na | 0,1 | 0,1 | 0,1 | 0,1 | 0,1 |
| Xanthan | 0,4 | 0,4 | 0,4 | 0,4 | 0,4 |
| Aroma | 1,2 | 1,2 | 1,2 | 1,2 | 1,2 |
| Ethyllauroylarginat | 0,15 | 0,25 | 0,35 | 0,45 | 0,8 |
| Kaliumnitrat | 5,0 | 5,0 | 5,0 | 5,0 | 5,0 |
| Wasser, vollentsalzt | ad 100 | ad 100 | ad 100 | ad 100 | ad 100 |

## Patentansprüche

1. Mund- und Zahnpflege- und -reinigungsmittel, enthaltend - bezogen auf sein Gewicht -
a) 0,01 bis 2,5 Gew.-% Ethyllauroylarginat
b) 0,1 bis 10,0 Gew.-% mindestens eines Kalium- oder Strontiumsalzes.

2. Mund- und Zahnpflege- und -reinigungsmittel nach Anspruch 1, **dadurch gekennzeichnet, daß** es - bezogen auf sein Gewicht - - 0,05 - 2,25 Gew.-%, vorzugsweise 0,075 - 2 Gew.-%, besonders bevorzugt 0,1 - 1,75 Gew.-%, außerordentlich bevorzugt 0,125 - 1,5 Gew.-% und insbesondere 0,15 bis 0,8 Gew.-% Ethyllauroylarginat enthält.

3. Mund und Zahnpflege- und -reinigungsmittel nach einem der Ansprüche 1 oder 2, **dadurch gekennzeichnet, daß** es 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e) oder Strontiumsalz(e) enthält.

4. Mund und Zahnpflege- und -reinigungsmittel nach einem der Ansprüche 1 bis 3, **dadurch gekennzeichnet, daß** es ausschließlich Kaliumsalz(e) enthält, welche(s) bei 20°C eine Löslichkeit in destilliertem Wasser oberhalb von 200 g/l, vorzugsweise oberhalb von 250 g/l und insbesondere oberhalb von 300 g/l aufweisen.

5. Mund und Zahnpflege- und -reinigungsmittel nach einem der Ansprüche 1 bis 4, **dadurch gekennzeichnet, daß** das Gewichtsverhältnis zwischen Ethyllauroylarginat und der Gesamtmenge an Kaliumsalz(en) 1 : 1000 bis 1 : 1, vorzugsweise 0,5 : 100 bis 75 : 100, weiter bevorzugt 1 : 100 bis 50 : 100,noch weiter bevorzugt 1,5 : 100 bis 10 : 100 und insbesondere 2 : 100 bis 3 : 100 beträgt.

6. Mund und Zahnpflege- und -reinigungsmittel nach einem der Ansprüche 1 bis 5, **dadurch gekennzeichnet, daß** es weniger als 60 Gew.-%, vorzugsweise weniger als 55 Gew.-%, besonders bevorzugt weniger als 50 Gew.-% und insbesondere weniger als 45 Gew.-% Wasser enthält.

7. Mund und Zahnpflege- und -reinigungsmittel nach einem der Ansprüche 1 bis 6, **dadurch gekennzeichnet, daß** es zur Verbesserung der entzündungshemmenden Eigenschaften eine Kombination von
- Panthenol oder einem Salz der Pantothensäure und
- Retinol oder einem Derivat davon
enthält.

8. Mund- und Zahnpflege- und -reinigungsmittel nach einem der Ansprüche 1 bis 7, **dadurch gekennzeichnet, daß** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthält.

9. Verwendung eines Mund und Zahnpflege- und -reinigungsmittels nach einem der Ansprüche 1 bis 8 zur
- Senkung der Zahnempfindlichkeit und/oder
- Senkung der Zahnempfindlichkeit gegen süße Speisen und Getränke und/oder
- Senkung der Zahnempfindlichkeit gegen saure Speisen und Getränke und/oder
- Senkung der Zahnempfindlichkeit gegen heiße Speisen und Getränke und/oder
- Senkung der Zahnempfindlichkeit gegen kalte Speisen und Getränke und/oder
- Senkung der Zahnempfindlichkeit gegen taktile Reize.

10. Verfahren zur Desensibilisierung empfindlicher Zähne, **dadurch gekennzeichnet, daß** ein Mund und Zahnpflege- und -reinigungsmittel nach einem der Ansprüche 1 bis 8 in Form einer Zahnpasta konfektioniert und zum Putzen der Zähne mittels einer Zahnbürste eingesetzt wird oder in Form eines Mundwassers konfektioniert ist und zum Spülen der Mundhöhle verwendet wird..
